# EUROPEAN PATENT SPECIFICATION

(11) **EP 4 658 188 B1**
(45) Date of publication and mention of the grant of the patent: **20.05.2026**
(21) Application number: 24715681.3
(22) Date of filing: 05.03.2024
(51) Int. Cl.: A61B 17/32, A61B 17/3209, A61B 17/22

(54) **HEART VALVE TISSUE CUTTING DEVICE**
HERZKLAPPENGEWEBESCHNEIDVORRICHTUNG
DISPOSITIF DE COUPE DE TISSU DE VALVULE CARDIAQUE

(30) Priority: 09.03.2023 US 202363489196 P
(43) Date of publication of application: 10.12.2025
(73) Proprietor: Pi-Cardia Ltd., 7632805 Rehovot (IL)
(72) Inventor: SPECTOR, Ben Zion, 7032019 Be'er Yaakov (IL)
(74) Representative: Pearl Cohen UK
(86) International application number: PCT/IB2024/052121
(87) International publication number: WO 2024/184809

(56) References cited:
- US-A1- 2020 289 102
- US-A1- 2022 313 303
- US-A1- 2022 346 827

## Description

### FIELD OF THE INVENTION

The present invention generally relates to devices for transcatheter modification of body tissue such as heart valve leaflets tissue like, e.g., aortic valve leaflets.

### BACKGROUND OF THE INVENTION

PCT Patent Application PCT/IB2020/054729 describes a transcatheter valve laceration device and method. The disclosure is a method and device, which can be used to perform BASILICA (Bioprosthetic or native Aortic Scallop Intentional Laceration to prevent Iatrogenic Coronary Artery obstruction), or a LAMPOON procedure (for mitral). The device is a cutting or splitting device with attention to preventing damage to neighboring tissues. The device can be implemented in other cardiologic procedures, such as tricuspidization of a bicuspid valve (turning a bicuspid valve into a tricuspid valve by cutting or splitting one of the bicuspid leaflets into two leaflets) or tricuspidization of a quadricuspid valve (lacerating one of the leaflets to turn the valve into a tricuspid valve) or splitting AML (anterior mitral leaflet) to prevent LVOTO (left ventricle outflow tract obstruction), thereby preparing the patient for safe transcatheter aortic or mitral valve replacement (TAVR/TMVR), or for other procedures that involve modifying cardiac /blood vessel tissue.
US-A1-2022/313303 describes a transcatheter valve laceration device including a leaflet support frame and a leaflet cutting assembly, both of which are movably mounted on a guiding structure and movable between contracted and expanded orientations.
US-A1-2020/289102 describes a device for treating damaged or diseased valves. An embodiment includes an elongated shaft having a longitudinal axis, a proximal portion, and a distal portion, and a dissection arm at the distal portion.
US-A1-2022/346827 describes a device for capturing and cutting fibrous and trabeculated structures (such as synechiae) in vessel lumens. In one embodiment, an intraluminal tissue modifying system configured to capture the fibrous structures, put the fibrous structures in tension, and controllably cut through the fibrous structures without applying appreciable additional force to the vessel wall.

### SUMMARY

The invention is defined by independent claim 1, with further embodiments defined by the dependent claims. The present invention seeks to provide a device for cutting heart valve tissue, such as but not limited to, aortic valve tissue, as is described more in detail hereinbelow. For example, the invention may be used in place of an electrified guidewire (which is the cutter used in prior art BASILICA) to traverse and lacerate the aortic leaflet down the center line.

The device of the invention is particular useful for lacerating the aortic valve leaflets by delivery via the aorta; however, the invention can be used to cut any heart tissue from various approaches and for cutting heart tissue at other valves or portions of the heart.

The term "cutting" refers to any kind of reduction in size or any modification in shape or form, such as but not limited to, cutting, splitting, lacerating, slicing, fracturing, chopping and the like, and the terms are used interchangeably throughout.

There is thus provided in accordance with the invention a heart valve tissue cutting device including a housing formed with an elongate opening, a cutting guide arm pivoted to the housing by a hinge assembly, the cutting guide arm being formed with a slot, an actuator wire coupled to the cutting guide arm, wherein movement of the actuator wire causes the cutting guide arm to pivot outwards away from the housing, a cutting element including a blade with a sharp tip, the cutting element being coupled to a track link which is arranged to travel along a track in the housing, and an activation slider coupled to a slender element and movable in a passageway in the housing, wherein movement of the activation slider causes the track link and the cutting element to move along the track, and wherein when the track link is at one end of the track, the blade does not protrude out of the housing and when the track link is not at the end of the track, the blade protrudes out of the housing.

In accordance with a non-limiting embodiment of the invention the hinge assembly includes a four-hinge mechanism.

In accordance with a non-limiting embodiment of the invention a blade protector rib is pivoted to the cutting guide arm, and wherein the four-hinge mechanism includes a first hinge link pivoted to a first portion of the housing at a first hinge and pivoted to the blade protector rib at a second hinge, and wherein a second hinge link is pivoted to a second portion of the housing at a third hinge and pivoted to the cutting guide arm at a fourth hinge.

In accordance with a non-limiting embodiment of the invention when the cutting guide arm is pivoted outwards, a portion of the four-hinge mechanism passes through the slot.

In accordance with a non-limiting embodiment of the invention the housing includes an end cap which limits movement of the four-hinge mechanism and defines a limit of outward pivoting movement of the cutting guide arm.

### BRIEF DESCRIPTION OF DRAWINGS

The present invention will be understood and appreciated more fully from the following detailed description, taken in conjunction with the drawings in which:
Fig. 1A is a simplified side view illustration of a heart valve tissue cutting device, in accordance with a non-limiting embodiment of the present invention.
Fig. 1B is a simplified top view illustration of the heart valve tissue cutting device.
Fig. 1C is a simplified bottom view illustration of the heart valve tissue cutting device.
Fig. 2A is simplified side view illustration of the heart valve tissue cutting device, including a cutting guide arm pivoted outwards, the cutting guide arm being formed with a slot for guiding the cutting action of a cutting element.
Fig. 2B is simplified side view illustration like Fig. 2A, showing internal components of the heart valve tissue cutting device, in particular showing a pivoting cutting element in a stowed position inside the cutting device and an activation slider coupled to the pivoting cutting assembly.
Fig. 3 is simplified side view illustration of deployment of the cutting assembly of the heart valve tissue cutting device, in which the activation slider has been moved from a first end of the device (e.g., the distal end) towards the second end of the device (such as, being moved proximally), so that the pivoting cutting element has been deployed outwards towards the cutting guide arm, so that the blade of the cutting element is about to enter the slot of the cutting guide arm.
Figs. 4A and 4B are simplified side view and lower perspective illustrations, respectively, in which the activation slider has been moved further towards the second end of the device so that the blade of the cutting element has entered the slot of the cutting guide arm.
Figs. 5A and 5B are simplified side view and upper perspective illustrations, respectively, in which the activation slider has been moved even further towards the second end of the device so that the axial travel of the blade of the cutting element can slice tissue.
Fig. 6 is a simplified side view illustration of the blade of the cutting element reaching its axial travel limit.
Figs. 7A and 7B are simplified side-view illustrations of the heart valve tissue cutting device, in accordance with another non-limiting embodiment of the present invention, including a grasping member for grasping a leaflet or other tissue prior to and during cutting, showing the grasping member in respective stowed and deployed positions.
Fig. 7C is a simplified side-view illustration of the heart valve tissue cutting device, in which the blade of the cutting element is deployed while the grasping member is in its deployed position.
Fig. 7D is a simplified perspective illustration of the heart valve tissue cutting device with the grasping member is in its deployed position.

### DETAILED DESCRIPTION

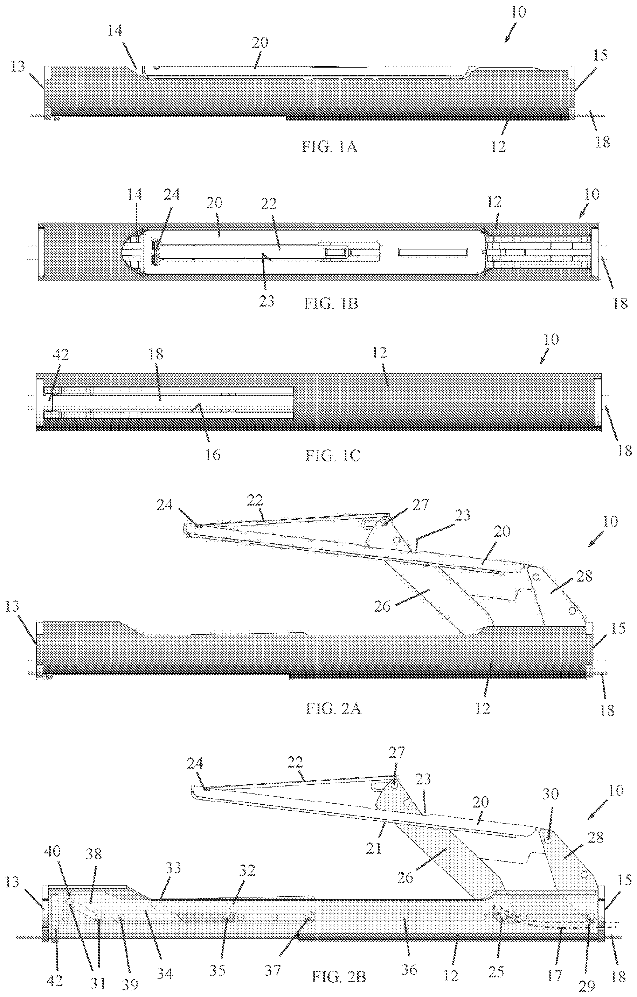

Reference is now made to Figs. 1A-1C, which illustrate a heart valve tissue cutting device 10, in accordance with a non-limiting embodiment of the present invention.

The heart valve tissue cutting device is particularly useful for cutting aortic valve tissue (such as aortic valve leaflets) and can be delivered via the aorta to cut the aortic valve tissue by initially stabbing or puncturing the tissue with a sharp pointed tip of the blade positioned at the distal end of the device, and then axially slicing the tissue by moving the blade towards the proximal end (in the direction back to the aorta). For such an approach, the distal end is the left end in the drawings and the proximal end is the right end in the drawings. It should be understood that distal and proximal are relative, non-limiting terms, and the invention can be used in other orientations. The invention is not limited to the aortic valve leaflets. The device of the invention can be implemented in other cardiologic procedures, such as cutting or slicing mitral valve leaflets, tricuspidization of a bicuspid valve (turning a bicuspid valve into a tricuspid valve by cutting or splitting one of the bicuspid leaflets into two leaflets) or tricuspidization of a quadricuspid valve (lacerating one of the leaflets to turn the valve into a tricuspid valve) or splitting AML (anterior mitral leaflet) to prevent LVOTO (left ventricle outflow tract obstruction), thereby preparing the patient for safe transcatheter aortic or mitral valve replacement (TAVR/TMVR), or for other procedures that involve modifying cardiac /blood vessel tissue.

The heart valve tissue cutting device 10 includes a (e.g., slender, cylindrical) housing 12 formed with an elongate opening 14, which may have rounded ends. The housing 12 may be made of medical grade stainless steel or any other suitable material.

As seen best in Fig. 1C, housing 12 may include a guidewire lumen 16 along a lower axial length of housing 12 ("lower" in the sense of the drawings; not necessarily lower when the device is being used). Accordingly, device 10 can be delivered over a guidewire 18 that passes though guidewire lumen 16. The housing 12 may include first and second end caps 13 and 15 (Fig. 1A).

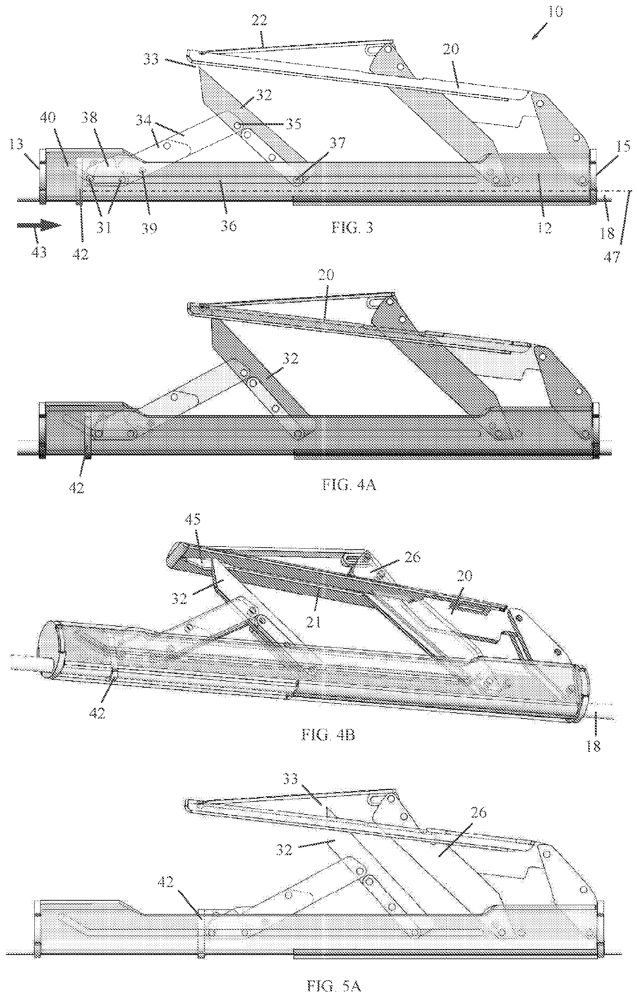

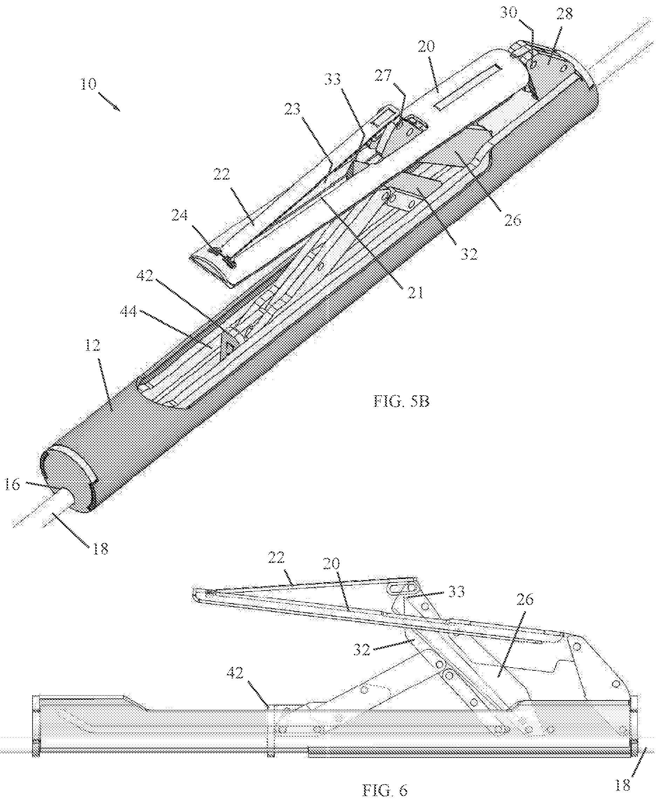

The heart valve tissue cutting device 10 includes a cutting guide arm 20 (Figs. 1A and 1B) pivoted to housing 12 by a hinge assembly (as described below with reference to Figs. 2A and 2B). A blade protector rib 22 may be pivoted to cutting guide arm 20 at a pivot or hinge 24 (Fig. 1B). In the contracted orientation of Figs. 1A, 1B, and 1C, blade protector rib 22 is located in an elongate rib aperture 23 (Fig. 1B and seen best in Fig. 5B) formed in cutting guide arm 20. The cutting guide arm 20 is formed with a slot 21 (seen in Fig. 4B) for guiding the cutting action of a cutting element, described hereinbelow.

Reference is now made to Figs. 2A and 2B. The cutting guide arm 20 may be pivoted to housing 12 by means of a four-hinge mechanism. A first hinge link 26 may be pivoted to a first portion of housing 12 at a first hinge (pivot) 25, and pivoted to blade protector rib 22 at a second hinge 27. Second hinge 27 may be a sliding hinge, in which a pin slides in a channel attached to blade protector rib 22. A second hinge link 28 may be pivoted to a second portion of housing 12 (proximal to the first portion), at a third hinge 29, which is proximal to first hinge 25, and pivoted to cutting guide arm 20 at a fourth hinge 30. In the orientation of Figs. 2A and 2B, in which cutting guide arm 20 is pivoted outwards, a portion of the four-hinge mechanism, first hinge link 26, passes through elongate rib aperture 23 (as seen in Fig. 5B) and through slot 21 (as seen in Fig. 4B). Slot 21 is formed on a portion of cutting guide arm 20 below elongate rib aperture 23 (thus elongate rib aperture 23 is not visible in Fig. 4B; again "below" is a relative term in the sense of the drawings). Second end cap 15 serves as a (proximal) stop for second hinge link 28 and defines the limit of the expanded position of cutting guide arm 20.

As seen in Fig. 2B, an actuator wire 17 may be provided for pivoting first hinge link 26 about first hinge (pivot) 25 for deploying cutting guide arm 20 outwards. Pulling actuator wire 17 proximally causes cutting guide arm 20 to pivot about first hinge 25 and third hinge 29. Alternatively, a biasing device (such as a coil or leaf spring) may be used to spring-load cutting guide arm 20 so that it can pivot about the hinges.

Reference is now made to Fig. 3, which illustrates deployment of a pivoting cutting assembly. The pivoting cutting assembly includes a cutting blade 32 which has a sharp, pointed tip 33. One portion of the cutting blade 32 may be pivoted to a blade support arm 34 at a first pivot (hinge) 35 and another portion of cutting blade 32 may be pivoted to a track 36 at a second pivot 37. Track 36 extends axially along a longitudinal portion of housing 12. The blade support arm 34 may be coupled to a track link 38, such as by a pin or rivet 39. (Alternatively, they could be one piece.) The track link 38 is arranged to travel along track 36 by means of one or pins 31 that are slidingly received in track 36. In the position of Fig. 2B, the track link 38 is on a distal, curved portion 40 of track 36 so that the cutting blade 32 is stowed and does not protrude outwards. The proximal curved portion 40 of track 36 curves from a central portion of housing 12 towards an outer contour of housing 12.

An activation slider 42 is arranged to move axially along guidewire 18 (the bottom of activation slider 42 is seen in Fig. 1C). The activation slider 42 is coupled to a wire or other slender element 47 (shown in Fig. 3) to move the activation slider 42 along guidewire 18. The slender element may be used for pulling and pushing; alternatively, it could be used just for pulling and a biasing device, such as a coil spring that gets compressed by proximal movement of the activation slider 42, could be provided for returning the activation slider to its initial position.

In the orientation of Fig. 2B, the activation slider 42 is adjacent first end cap 13. Activation slider 42 abuts against the track link 38. Activation slider 42 may be oblong and slide in a passageway 44 (seen in Fig. 5B) which is complementarily shaped to accommodate the shape of activation slider 42 to guide the axial movement of activation slider 42. For example, the cross-sectional shape of passageway 44 may be oblong to match the oblong shape of activation slider 42, with sufficient tolerance to allow for easy and smooth movement of activation slider 42.

In the orientation of Fig. 3, the activation slider 42 has been moved from a first end of the device (e.g., the distal end) towards the second end of the device (such as, being moved proximally as indicated by arrow 43), so that the pivoting cutting blade 32 has been deployed outwards towards the cutting guide arm 20, so that the blade 32 is about to enter the slot of cutting guide arm 20. In the position of Fig. 3, the activation slider 42 has pushed track link 38 proximally away from the distal, curved portion 40 of track 36, which causes blade support arm 34 to move outwards to deploy cutting blade 32 outwards. The track link 38 now slides on the straight portion of track 36.

In the orientation of Fig. 3, the sharp tip 33 of blade 32 can pierce (stab) heart valve tissue which is located between the blade 32 and cutting guide arm 20.

Reference is now made to Figs. 4A and 4B. Activation slider 42 has been moved further towards the second end of the device so that blade 32 has entered slot 21 (|Fig. 4B) of cutting guide arm 20. Slot 21 will now guide the axial slicing action of blade 32 as the blade moves proximally. It is seen in Fig. 4B, slot 21 may be formed with a widened portion 45 (which may be diamond shaped or have other shapes), at a distal portion thereof. The widened portion 45 accommodates for any swerving of the blade at the initial stabbing point and allows the blade to smoothly enter the rest of slot 21.

Reference is now made to Figs. 5A and 5B. Activation slider 42 has been moved even further towards the second end of the device so that the axial travel of blade 32 can slice tissue.

Reference is now made to Fig. 6, which illustrates blade 32 reaching its axial travel limit at first hinge link 26. At this limit, first hinge link 26 serves as an anvil to ensure that blade 32 cuts properly through the full thickness of the leaflet or other tissue (the cutting action against this "anvil" is like the final cut in a guillotine or paper cutter).

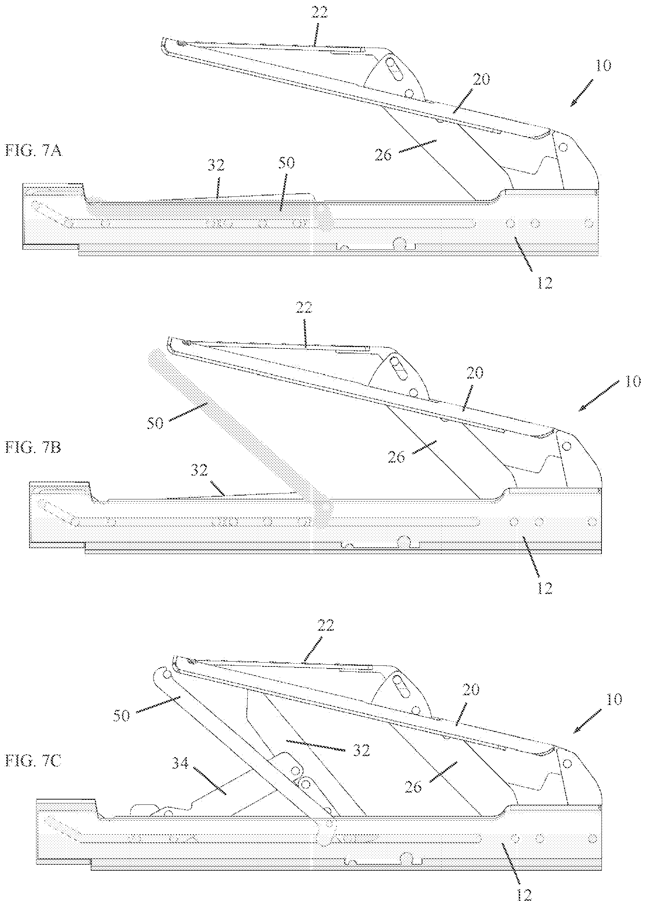

Reference is now made to Figs. 7A-7D, which illustrate heart valve tissue cutting device 10, in accordance with another non-limiting embodiment of the present invention. This embodiment includes a grasping member 50 for grasping a leaflet or other tissue prior to and during cutting. The grasping member 50 may be spring-loaded or wire activated, similarly as described hereinabove for deploying cutting guide arm 20.

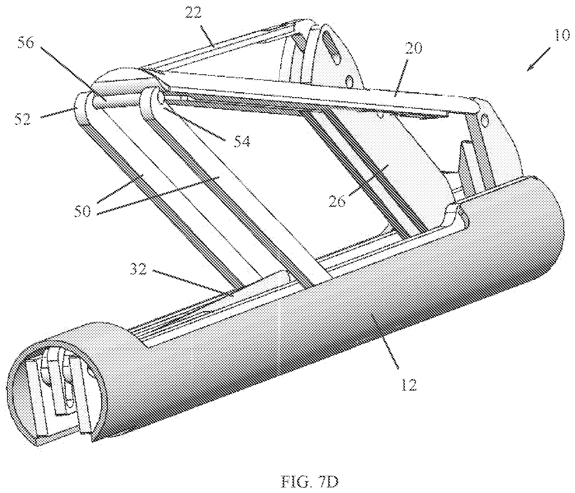

As seen best in Fig. 7D, grasping member 50 may include a pair of arms whose ends 52 and 54 are connected to each other with a bridge member 56. After positioning the cutting guide arm 20 over the desired position on the leaflet or other tissue to be cut or sliced, the grasping member 50 may be raised to hold the leaflet or other tissue in place (sandwiched in place), while blade 32 is activated.

## Claims

1. A heart valve tissue cutting device (10) comprising:
a housing (12) formed with an elongate opening (14);
a cutting guide arm (20) pivoted to said housing (12) by a hinge assembly, said cutting guide arm (20) being formed with a slot (21);
an actuator wire (17) coupled to said cutting guide arm (20), wherein movement of said actuator wire (17) causes said cutting guide arm (20) to pivot outwards away from said housing (12);
a cutting element comprising a blade (32) with a sharp tip (33), said cutting element being coupled to a track link (38) which is arranged to travel along a track (36) in said housing (12); and
an activation slider (42) coupled to a slender element (47) and movable in a passageway (44) in said housing (12), wherein movement of said activation slider (42) causes said track link (38) and said cutting element to move along said track (36), and wherein when said track link (38) is at one end of said track (36), said blade (32) does not protrude out of said housing (12) and when said track link (38) is not at said end of said track (36), said blade (32) protrudes out of said housing (12).

2. The heart valve tissue cutting device (10) according to claim 1, wherein said hinge assembly comprises a four-hinge mechanism.

3. The heart valve tissue cutting device (10) according to claim 2, wherein a blade protector rib (22) is pivoted to said cutting guide arm (20), and wherein said four-hinge mechanism comprises a first hinge link (26) pivoted to a first portion of said housing (12) at a first hinge (25) and pivoted to said blade protector rib (22) at a second hinge (27), and wherein a second hinge link (27) is pivoted to a second portion of said housing (12) at a third hinge (29) and pivoted to said cutting guide arm (20) at a fourth hinge (30).

4. The heart valve tissue cutting device (10) according to claim 3, wherein when said cutting guide arm (20) is pivoted outwards, a portion of said four-hinge mechanism passes through said slot (21).

5. The heart valve tissue cutting device (10) according to claim 2, wherein said housing (12) comprises an end cap (15) which limits movement of said four-hinge mechanism and defines a limit of outward pivoting movement of said cutting guide arm (20).

6. The heart valve tissue cutting device (10) according to claim 3, wherein said second hinge is a sliding hinge, in which a pin slides in a channel attached to said blade protector rib (22).

7. The heart valve tissue cutting device (10) according to any one of claims 1-6, wherein said end of said track (36) at which said blade (32) does not protrude out of said housing (12) comprises a curved portion (40) of said track (36) that curves from a central portion of said housing (12) towards an outer contour of said housing (12).

8. The heart valve tissue cutting device (10) according to any one of claims 1-7, wherein said blade (32) is pivoted to a blade support arm (34) at a first pivot (35) and another portion of said blade is pivoted to said track (36) at a second pivot (37), and said blade support arm (34) is coupled to said track link (38).

9. The heart valve tissue cutting device (10) according to any one of claims 1-8, wherein during movement of said cutting element along said track (36), said blade (32) is configured to move through said slot (21).

10. The heart valve tissue cutting device (10) according to any one of claims 1-9, further comprising a grasping member (50) for grasping a leaflet or other tissue prior to and during cutting.

## Patentansprüche

1. Schneidvorrichtung (10) für Herzklappengewebe, umfassend:
ein Gehäuse (12), das mit einer langgestreckten Öffnung (14) ausgebildet ist;
einen Schneidführungsarm (20), der durch eine Gelenkanordnung schwenkbar mit dem Gehäuse (12) verbunden ist, wobei der Schneidführungsarm (20) mit einem Schlitz (21) ausgebildet ist;
einen Betätigungsdraht (17), der an den Schneidführungsarm (20) gekoppelt ist, wobei Bewegung des Betätigungsdrahts (17) bewirkt, dass der Schneidführungsarm (20) von dem Gehäuse (12) weg nach außen schwenkt;
ein Schneidelement, das eine Klinge (32) mit einer scharfen Spitze (33) umfasst, wobei das Schneidelement an ein Schienenglied (38) gekoppelt ist, das dazu eingerichtet ist, sich entlang einer Schiene (36) in dem Gehäuse (12) voranzubewegen; und
einen Aktivierungsschieber (42), der an ein schlankes Element (47) gekoppelt ist und in einem Durchgang (44) in dem Gehäuse (12) bewegbar ist, wobei Bewegung des Aktivierungsschiebers (42) bewirkt, dass sich das Schienenglied (38) und das Schneidelement entlang der Schiene (36) bewegen, und wobei, wenn sich das Schienenglied (38) an einem Ende der Schiene (36) befindet, die Klinge (32) nicht aus dem Gehäuse (12) ragt, und wenn sich das Schienenglied (38) nicht an dem Ende der Schiene (36) befindet, die Klinge (32) aus dem Gehäuse (12) ragt.

2. Schneidvorrichtung (10) für Herzklappengewebe nach Anspruch 1, wobei die Gelenkanordnung einen Viergelenkmechanismus umfasst.

3. Schneidvorrichtung (10) für Herzklappengewebe nach Anspruch 2, wobei eine Klingenschutzrippe (22) mit dem Schneidführungsarm (20) schwenkbar verbunden ist und wobei der Viergelenkmechanismus ein erstes Gelenkglied (26) umfasst, das an einem ersten Gelenk (25) mit einem ersten Abschnitt des Gehäuses (12) schwenkbar verbunden ist und an einem zweiten Gelenk (27) mit der Klingenschutzrippe (22) schwenkbar verbunden ist, und wobei ein zweites Gelenkglied (27) an einem dritten Gelenk (29) mit einem zweiten Abschnitt des Gehäuses (12) schwenkbar verbunden ist und an einem vierten Gelenk (30) mit dem Schneidführungsarm (20) schwenkbar verbunden ist.

4. Schneidvorrichtung (10) für Herzklappengewebe nach Anspruch 3, wobei, wenn der Schneidführungsarm (20) nach außen geschwenkt wird, ein Abschnitt des Viergelenkmechanismus durch den Schlitz (21) hindurch gelangt.

5. Schneidvorrichtung (10) für Herzklappengewebe nach Anspruch 2, wobei das Gehäuse (12) eine Endkappe (15) umfasst, die Bewegung des Viergelenkmechanismus begrenzt und eine Grenze der Schwenkbewegung nach außen des Schneidführungsarms (20) definiert.

6. Schneidvorrichtung (10) für Herzklappengewebe nach Anspruch 3, wobei das zweite Gelenk ein Gleitgelenk ist, in dem ein Zapfen in einer an der Klingenschutzrippe (22) angebrachten Führung gleitet.

7. Schneidvorrichtung (10) für Herzklappengewebe nach einem der Ansprüche 1-6, wobei das Ende der Schiene (36), an dem die Klinge (32) nicht aus dem Gehäuse (12) ragt, einen gekrümmten Abschnitt (40) der Schiene (36) umfasst, der von einem mittleren Abschnitt des Gehäuses (12) in Richtung eines äußeren Umrisses des Gehäuses (12) gekrümmt ist.

8. Schneidvorrichtung (10) für Herzklappengewebe nach einem der Ansprüche 1-7, wobei die Klinge (32) an einem ersten Drehpunkt (35) mit einem Klingenstützarm (34) schwenkbar verbunden ist und ein anderer Abschnitt der Klinge an einem zweiten Drehpunkt (37) mit der Schiene (36) schwenkbar verbunden ist, und der Klingenstützarm an das Schienenglied (38) gekoppelt ist.

9. Schneidvorrichtung (10) für Herzklappengewebe nach einem der Ansprüche 1-8, wobei, während Bewegung des Schneidelements entlang der Schiene (36), die Klinge (32) dazu konfiguriert ist, sich durch den Schlitz (21) zu bewegen.

10. Schneidvorrichtung (10) für Herzklappengewebe nach einem der Ansprüche 1-9, ferner umfassend ein Greifelement (50) zum Greifen eines Segels oder anderen Gewebes vor und während des Schneidens.

## Revendications

1. Dispositif de coupe de tissu de valve cardiaque (10) comprenant :
un logement (12) formé avec une ouverture allongée (14) ;
un bras de guide de coupe (20) relié de manière pivotante audit logement (12) par un ensemble de charnière, ledit bras de guide de coupe (20) étant formé avec une fente (21) ;
un fil d'actionneur (17) couplé audit bras de guide de coupe (20), dans lequel le mouvement dudit fil d'actionneur (17) amène ledit bras de guide de coupe (20) à pivoter vers l'extérieur à l'écart dudit logement (12) ;
un élément de coupe comprenant une lame (32) avec une pointe tranchante (33), ledit élément de coupe étant couplé à une liaison de voie (38) qui est agencée pour se déplacer le long d'une voie (36) dans ledit logement (12) ; et
un curseur d'activation (42) couplé à un élément mince (47) et capable de mouvement dans un passage (44) dans ledit logement (12), dans lequel le mouvement dudit curseur d'activation (42) amène ladite liaison de voie (38) et ledit élément de coupe à se déplacer le long de ladite voie (36), et dans lequel lorsque ladite liaison de voie (38) se trouve à une extrémité de ladite voie (36), ladite lame (32) ne fait pas saillie hors dudit logement (12) et lorsque ladite liaison de voie (38) ne se trouve pas au niveau de ladite extrémité de ladite voie (36), ladite lame (32) fait saillie hors dudit logement (12).

2. Dispositif de coupe de tissu de valve cardiaque (10) selon la revendication 1, dans lequel ledit ensemble de charnière comprend un mécanisme à quatre charnières.

3. Dispositif de coupe de tissu de valve cardiaque (10) selon la revendication 2, dans lequel une nervure de protecteur de lame (22) est reliée de manière pivotante audit bras de guide de coupe (20), et dans lequel ledit mécanisme à quatre charnières comprend une première liaison de charnière (26) reliée de manière pivotante à une première partie dudit logement (12) au niveau d'une première charnière (25) et reliée de manière pivotante à ladite nervure de protecteur de lame (22) au niveau d'une deuxième charnière (27), et dans lequel une deuxième liaison de charnière (27) est reliée de manière pivotante à une deuxième partie dudit logement (12) au niveau d'une troisième charnière (29) et reliée de manière pivotante audit bras de guide de coupe (20) au niveau d'une quatrième charnière (30).

4. Dispositif de coupe de tissu de valve cardiaque (10) selon la revendication 3, dans lequel, lorsque ledit bras de guide de coupe (20) est pivoté vers l'extérieur, une partie dudit mécanisme à quatre charnières passe à travers ladite fente (21).

5. Dispositif de coupe de tissu de valve cardiaque (10) selon la revendication 2, dans lequel ledit logement (12) comprend un capuchon d'extrémité (15) qui limite le mouvement dudit mécanisme à quatre charnières et définit une limite de mouvement de pivotement vers l'extérieur dudit bras de guide de coupe (20).

6. Dispositif de coupe de tissu de valve cardiaque (10) selon la revendication 3, dans lequel ladite deuxième charnière est une charnière coulissante, dans laquelle une broche coulisse dans un canal attaché à ladite nervure de protecteur de lame (22).

7. Dispositif de coupe de tissu de valve cardiaque (10) selon l'une quelconque des revendications 1 à 6, dans lequel ladite extrémité de ladite voie (36) au niveau de laquelle ladite lame (32) ne fait pas saillie hors dudit logement (12) comprend une partie incurvée (40) de ladite voie (36) qui s'incurve à partir d'une partie centrale dudit logement (12) en direction d'un contour externe dudit logement (12).

8. Dispositif de coupe de tissu de valve cardiaque (10) selon l'une quelconque des revendications 1 à 7, dans lequel ladite lame (32) est reliée de manière pivotante à un bras de support de lame (34) au niveau d'un premier pivot (35) et une autre partie de ladite lame est reliée de manière pivotante à ladite voie (36) au niveau d'un deuxième pivot (37), et ledit bras de support de lame (34) est couplé à ladite liaison de voie (38).

9. Dispositif de coupe de tissu de valve cardiaque (10) selon l'une quelconque des revendications 1 à 8, dans lequel pendant le mouvement dudit élément de coupe le long de ladite voie (36), ladite lame (32) est configurée pour se déplacer à travers ladite fente (21).

10. Dispositif de coupe de tissu de valve cardiaque (10) selon l'une quelconque des revendications 1 à 9, comprenant en outre un élément de préhension (50) pour saisir un feuillet ou un autre tissu avant et pendant la coupe.
